# EUROPEAN PATENT SPECIFICATION

(11) **EP 3 718 579 B1**
(45) Date of publication and mention of the grant of the patent: **25.12.2024**
(21) Application number: 20175612.9
(22) Date of filing: 23.07.2010
(51) Int. Cl.: A61L 27/12, A61L 27/46, A61L 27/50, A61L 27/56, A61L 27/58

(54) **INJECTABLE AND MOLDABLE OSTEOINDUCTIVE CERAMIC MATERIALS**
INJIZIERBARE UND FORMBARE OSTEOINDUKTIVE KERAMIKMATERIALIEN
MATÉRIAUX CÉRAMIQUES OSTÉOINDUCTIFS INJECTABLES ET MOULABLES

(30) Priority: 23.07.2009 WO PCT/NL2009/050459
(43) Date of publication of application: 07.10.2020
(62) Divisional of application: 10740529.2
(73) Proprietor: NuVasive Netherlands B.V., 1081 KM Amsterdam (NL)
(72) Inventor: YUAN, Huipin, 3704 VK Zeist (NL); BARBIERI, Davide, 3702 CB Zeist (NL); DE BRUIJN, Joost Dick, 3817 JL Amersfoort (NL); DE GROOT, Florence, 3583 SJ Utrecht (NL); DAVISON, Noel, 3514 BT Utrecht (NL)
(74) Representative: V.O.

(56) References cited:
- EP-A1- 0 987 032
- WO-A1-2007/094672
- US-A- 5 626 861
- US-A1- 2004 002 770
- US-A1- 2006 172 918
- NIHOUANNEN, D ET AL.: "Bone tissue formation in sheep muscles induced by a biphasic calcium phosphate ceramic and fibrin glue composite", JOURNAL OF MATERIALS SCIENCE: MATERIALS IN MEDICINE, vol. 19, no. 2, February 2008 (2008-02-01), KLUWER ACADEMIC PUBLISHERS, BO, pages 667 - 675, XP019575620, ISSN: 1573-4838
- YOKOZEKI H ET AL: "Influence of surface microstructure on the reaction of the active ceramics in vivo", JOURNAL OF MATERIALS SCIENCE. MATERIALS IN MEDICINE, CHAPMAN AND HALL, LONDON, GB, vol. 9, no. 7, 1 July 1998 (1998-07-01), pages 381 - 384, XP002093992, ISSN: 0957-4530
- HABIBOVIC, P ET AL.: "3D microenvironment as essential element for osteoinduction by biomaterials", BIOMATERIALS, vol. 26, 1 June 2005 (2005-06-01), ELSEVIER SCIENCE PUBLISHERS BV., BARKING, GB, pages 3565 - 3575, XP004696105, ISSN: 0142-9612

## Description

### FIELD OF THE INVENTION

The invention refers to bioactive ceramic materials, in particular calcium phosphates, demonstrating osteoinductive properties on the basis of, amongst others, their surface microstructure, and in particular their surface microporosity. This particular class of calcium phosphates is hereinafter referred to as surface-microstructured, and particularly surface-microporous calcium phosphates. The invention refers to osteoinductive ceramic materials, preferably that are injectable and/or moldable.

### BACKGROUND OF THE INVENTION

The development of ceramic materials, which is preferably osteoinductive, is an important advancement in those areas of medicine dealing with the treatment of osseous defects. This aids in minimizing the need for harvesting autologous bone from a patient's own bone, as harvestable autologous bone is scarce. Osteoinductive materials are capable of inducing the development of new bone tissue. In general, such bone-induction is defined as the mechanism by which a mesenchymal tissue is induced to change its cellular structure to become osteogenic.

A background reference to osteoinductivity exhibited by porous calcium phosphates is Yamasaki et al. in Biomaterials 13:308-312 (1992). These materials can be characterized as porous calcium phosphates exhibiting surface microporosity. Since then, as a major advancement as compared to the materials disclosed by Yamasaki, porous calcium phosphate materials have become available that show improved osteoinductive properties. The surface microstructured, and specifically surface microporous calcium phosphates of the present invention demonstrate a wider range of micropore and granule size, as well as high total porosity.

One representative reference to osteoinductive, surface-microporous calcium phosphates, is US 6,511 ,510. Another representative reference, directed to even further improved osteoinductive, surface-microporous calcium phosphates, is WO 2007/94672.

Another improvement in the treatment of osseous defects, resides in the incorporation of materials having an activity contributing to the formation of bone, in pasty materials, such as pastes, gels, putties, or the like. A background reference in this respect is, e.g., WO 2007/068489. Herein a paste material is employed to form, together with a particulate solid porous material a matrix usable for the replacement or augmentation of bone. The particulate solid porous material can be calcium phosphate. The result is a macroporous scaffold for bone growth, preferably provided with active components such as cells, growth factors or bone-induction agents. Another background reference to injectable formulations for filling bone is WO 2003/028779. Herein a bone filler comprising calcium salt particles is provided with an organic binder, and cells such as stem cells, osteogenic cells, and osteoprogenitor cells. Neither of these references relates to the use of porous calcium phosphates that are osteoinductive per se and the described calcium phosphate materials are not of the aforementioned surface-microstructured, particularly surface- microporous type. Rather, if the systems described in these references were used on the aforementioned class of osteoinductive surface-microporous calcium phosphates, the paste material of WO 2007/068489, respectively the binder of WO 2003/028779, would cover the surface of the calcium phosphate. US 2004/002770 A1 refers to a polymer-bioceramic composite comprising macropores or micropores, wherein the pores are optionally interconnected, for use in repair of bone defects. Obviously, it is counter-intuitive to select a technology that covers a surface, if the surface structure of the material is key to its intended function. The problem underlying the present invention is the development of materials, in particular comprising calcium phosphate, which have improved osteoinductive effects and possess the advantages of being moldable and/or injectable.

### SUMMARY OF THE INVENTION

The present invention refers to a biomaterial comprising porous calcium phosphate, which is osteoinductive, having a surface-microstructure, and particularly a surface-microporosity, and a carrier, which is a water-free blend of polymers that disintegrates under physiological circumstances, as defined in claim 1.

Also disclosed, is an osteoinductive biomaterial, comprising porous calcium phosphate having surface-microporosity, and a carrier wherein the carrier disintegrates, in vivo, within a predefined time period for bone growth initiation, particularly within 6 weeks.

Also disclosed, is an osteoindcutive biomaterial, comprising porous calcium phosphate having surface-microporosity, and a carrier, wherein the carrier has preferably a dissolution time of one week in physiological saline at 37°C. In yet another aspect, the invention refers to a biomaterial, which is osteoinductive, comprising porous calcium phosphate having surface-microporosity, and a water-free carrier, wherein the carrier has a Dimensional Stability Life, which is the time period during which the polymer, in a human body temperature environment is not substantially dissolved or disassociated, of at most a week.

Also disclosed, is the use of water-free carriers, as mentioned hereinbefore, to aid in delivering and containing a surface-porous biomaterial, which is preferably osteoinductive, while preserving its osteoinductivity.

### DETAILED DESCRIPTION OF THE INVENTION

The invention is defined by the claims. Any subject-matter falling outside the scope of the claims is provided for information purposes only. Any references in the description to methods of treatment refer to ceramic materials of the present invention for use in a method for treatment or surgery of the human (or animal) body by therapy (or surgery).

Surface-microstructured (microporous) bioactive materials, preferably osteoinductive Previous studies have shown that certain materials can induce bone formation in ectopic, non-osseous sites such as muscle. The surface microstructure of the materials has shown to play an important role in this bone induction (osteoinduction) process. The current hypothesis for material-induced bone formation is that the adsorption of proteins and growth factors, followed by migration and attachment of stem- or progenitor cells and their subsequent proliferation and osteogenic differentiation, play an important role [H. Yuan et al, Biomaterials 20:1799-1806 (1999); P. Habibovic et al, Biomaterials 26:3565-3575 (2005)]. Osteoinductivity of materials, such as calcium phosphate, is defined as the property by which the materials induce ectopic bone formation in non-osseous sites without the addition of osteogenic cells or bone growth factors prior to implantation. In general, such bone induction is defined as the mechanism by which a mesenchymal tissue is induced to change its cellular function to become osteogenic.

The invention is directed to calcium phosphate materials, which are preferably osteoinductive, that have a surface microstructure, and particularly a surface microporosity. Microporosity is herein defined as pertaining to pores having a size below 1.5 µm as determined with mercury intrusion and scanning electron microscopy. Surface microstructure generally indicates that the surface comprises protrusions and / or indentations on a microscale, i.e. below 10 µm, preferably below 5 µm, and more preferably below 1.5 µm.

One way of obtaining calcium phosphate having surface microporosity is disclosed in US 6,511 ,510. Herein a (macro)porous material is provided, and subjected to an acid treatment to create micropores in the surface of the macropores. This material has a total porosity of 20 to 90%, wherein macropores are present having a size ranging from 0.1 to 1.5 mm, and wherein micropores are present having a size ranging from 0.05 to 20 µm.

The ceramic material is calcium phosphate. Preferred calcium phosphate is octacalcium phosphate, apatites, such as hydroxyapatite or carbonate apatite, whitlockites, such as [alpha]-tricalcium phosphate and [beta]-tricalcium phosphate, and combinations thereof.

An important aspect of these ceramic materials, which are osteoinductive, is the physical structure of the material, in particular a biomaterial. The material comprises both macropores and micropores. The total porosity ranges from 20 to 90%, preferably from 40 to 80%, and most preferably between 50 and 80 %. The macropores of the material have a size of from 0.1 to 1.5 mm; preferably, the size of the macropores lies between 0.2 and 1 mm. The size of the macropores has a significant beneficial influence for example on the osteoinductive character of the material, in particular if the macropores are interconnected. The micropores of the material have a size of from 0.05 to 20 µm. A preferred range for the size of the micropores is from 0.1 to 10 µm, 0.1 to 3 µm, or 0.1 to 5 µm, preferably 0.1 to 1.5 µm. The micropores are at least present in the surface of the macropores.

The microporosity of the material surface preferably lies between 20 and 60%, preferably between 30 and 50%. In accordance with US 6,511 ,510, the biomaterial preferably consists of crystals. Preferably, the size of the crystals is similar to the size of the micropores, as this results in preferable microrugosity of the biomaterial. Thus, the size of the crystals lies preferably between 0.05 and 20 µm, more preferably between 0.1 and 10 µm, 0.1 to 3 µm, or 0.1 to 5 µm, and most preferably between 0.1 and 1.5 µm

Another, and preferred, way of obtaining calcium phosphate having surface microporosity is disclosed in WO 2007/094672. This refers to a porous calcium phosphate material, which is preferably osteoinductive, having an average grain size in a range of 0.1 to 1.5 µm, a porosity comprising micropores in a size range of 0.1 to1.50 µm, and having a surface area percentage of micropores in a range of 10 to 40%. The surface area percentage of micropores preferably is below 40%, more preferably 1 to 30 % or 1 to 20 %, most preferably in a range from 10 to 25%.

A porous calcium phosphate of the present invention preferably has a protein adsorption capacity of at least 40%, preferably 40 to 100 %, 50 to 100 %, 60 to 100 %, 70 to100 %, 80 to 100 % or 90 to 100 %. The adsorption capacity is expressed as the percentage of protein absorbed by a volume of 1.0 ml of said calcium phosphate from a volume of 3 ml of a 1% aqueous solution of fetal bovine serum (FBS) in the presence of 25ppm sodium azide (NaN3) after 24 hrs at 37°C.

In the present invention, the surface-microstructured, particularly surface-microporous calcium phosphate is mixable with a carrier. To this end the calcium phosphate generally is in the form of particulate matter, preferably granular or a powder, either irregularly or regularly shaped, for example amorphous or simple geometric prisms like cubes, cuboids, spheres, or cylinders.

A porous calcium phosphate, which is osteoinductive, as used in the present invention is preferably in the form of microparticles having a particle size ranging from about 45 to about 1500 µm, more preferably from about 200 to about 300 µm, most preferably 45-106 µm, 106-212 µm, or 212-300 µm.

In another preferred embodiment of the invention, the calcium phosphate powder is TCP or BCP. BCP is a mixture of TCP and HA1 with HA constituting greater than 0% and less than 100% of the mixture, with granules larger than 45 µm.

In yet another preferred embodiment of the invention, the microparticles collected after milling of the sintered calcium phosphate are subsequently cleaned for example ultrasonically with acetone, ethanol and/or water, and optionally dried and sterilized.

In yet a further preferred embodiment of the invention, the calcium phosphate powder is an oven dried milled powder having particles of irregular shape.

With a view to obtaining optimal osteoinductive properties, the calcium phosphate will have a particle size of at least 45 µm. The preferred upper limit for the particle size is 4000 µm, although larger particle sizes are not excluded and are advantageous in terms of easier handling by the medical practitioner. The particle size is more preferably selected from the group consisting of 45 µm to 1000 µm, 45-500 µm, and more preferably 45-300 µm, 45 µm to 150 µm, 150 µm to 1000 µm, 150 µm to 500 µm, 500 µm to 1000 µm, 1000 µm to 2000 µm, 1000 µm to 4000 µm, and mixtures thereof. In a preferred embodiment, calcium phosphate of any of the indicated particle sizes is used for the preparation of a moldable and/or injectable preparation of the invention, i.e., for any injectable or putty.

The resulting ceramic materials, which are osteoinductive, made by combining calcium phosphate particles and a water-free carrier, as disclosed in the present invention are preferably in the form of putties or injectables. Putties are applied to the surgical site by hand or surgical instrument, and embody moldable, malleable, and/or kneadable handling properties. Injectable materials are flowable and extrudable through a standard or custom-made syringe nozzle by manual force or with the assistance of a mechanical dispensing device (e.g., a caulk gun), and may also be moldable upon extrusion.

Preferably, the force required to extrude an injectable ceramic material of the present invention is less than 100 N. In general terms, putties are characterized by their higher stiffness resulting in optimal moldability, whereas injectables are characterized by their lower stiffness and flowability resulting in optimal extrusion through a standard or custom- made syringe.

### Water-free Carrier

A water-free carrier is defined as a binder in any molecular form - for example as paste, gel, powder, or granular - that physically holds an active component. The use of a water- free carrier of the present invention allows an optimized handling and application of the active ingredient to the treatment site. For example, the ceramic material, which is osteoinductive, of the present invention is injectable during minimally invasive procedures, due to the flowable characteristics of the water-free carrier comprising the ceramic particles. In another example, the ceramic material of the present invention, which is osteoinductive, is a moldable putty which fits in complex shaped defects and allows long term retention of the active component at the treatment site due to the moldable and malleable characteristics imparted by the water-free carrier.

The water-free carrier of the present invention is used versus water-containing carriers (e.g., JAX Bone Void Filler^{®}, Smith and Nephew, comprising carboxymethyl cellulose aqueous gel) in order to better preserve the surface-microstructure of the ceramic particles, which are preferably osteoinductive. The nature of calcium phosphate ceramic's crystalline structure and chemistry makes it susceptible to degradation in aqueous environments. Thus, water-containing calcium phosphate materials demonstrate limited shelf-life, or must be inconveniently reconstituted or mixed in the surgical suite prior to implantation.

Moreover, if the calcium phosphate is microstructured, as in the present invention, this degradation may adversely affect its desired effect such as osteoinductivity. Therefore, water-free carriers contain and store calcium phosphate particles in order to prolong the shelf-life of such microstructured ceramic materials. Moreover, water-free carriers enable the convenience of a ready-to-use ceramic material, which is osteoinductive, without reconstitution or component mixing prior to implantation.

Fast dissolution time of the water-free carrier is also preferred so that the surface microstructure of the comprised calcium phosphate particles, which are osteoinductive, may be most expediently exposed to implantation environment, thereby affecting an osteoinductive response as soon as possible after implantation.

A water-free carrier, as defined in claim 1, comprises: 1) xanthan, dextran, starch and glycerol, or 2) CMC7H, PEG4k, PEG400 and glycerol, or 3) xanthan, dextran and Solutol HS15, or 4) xanthan and glycerol, or 5) CMC50k and glycerol, or 6) xanthan, Solutol HS15 and glycerol.

The water-free carrier is a water-free blend of polymers, providing a significantly longer shelf life of the comprised for example osteoinductive particles than would a comparable water comprising carrier, i.e., an aqueous polymer, for example a polymeric gel with a physiologically acceptable solvent, particularly water and/or physiological saline. The water-free carrier serves, as mentioned above, as a binder for calcium phosphate particles and is thus present upon administration (e.g. injection or implantation) of the calcium phosphate materials of the invention to a subject. The carrier is selected so as to allow the calcium phosphate material to retain its characteristic properties such as osteoinductivity.

The osteoinductivity is preferably retained if (i) the microstructure is preserved i.e. by pre-packing the particles, for example osteoinductive particles with a water-free carrier, and (ii) the water-free carrier is as defined in claim 1; that dissolve or disintegrate before for example bone formation takes place. Disintegration refers to the characteristic of a water-free carrier to dissolve, dissociate, or otherwise fall apart in any possible way after placement (e.g. via injection or manual implantation) into the human body. Here, dissolution is defined by the disentanglement and separation of polymer molecules in aqueous solution (i.e., saline or blood) and is evaluated visually with respect to time. The water-free carriers, for example specific for surface-microporous osteoinductive materials, are unprecedented in the art, e.g., in the aforementioned WO 2003/028779. A requirement is that the organic binder used therein does not disappear, i.e., dissolves or disassociates, until for example bone formation has taken place to a sufficient extent to take over the function of living bone.

Thus, essentially, the water-free carrier serves as delivery vehicle in the administration (introduction into the body by injection or implantation) of the surface-microporous materials, which is osteoinductive, but thereafter, it is due to disassociate by dissolution or other modes of disintegration, allowing for example active bone formation in situ.

In this respect, the invention also relates to the osteoinductive ceramic material for use in promoting bone growth in a subject in need thereof, by introducing into the subject's body a material, which is osteoinductive, wherein the material is combined with the biocompatible water- free carrier, as defined in claim 1, and wherein the water-free carrier is a processing aid that starts to dissolve or disassociate after introduction into the subject's body and before the onset of bone formation of the material. More particularly, the water-free carrier herein is substantially dissolved or disassociated before completion of bone formation and, preferably, before the onset of bone formation. The latter will allow the full benefit from the surface- microporosity of the present materials, which are preferably osteoinductive.

The water-free carriers as defined in claim 1, comprises: 1) xanthan, dextran, starch and glycerol, or 2) CMC7H, PEG4k, PEG400 and glycerol, or 3) xanthan, dextran and Solutol HS15, or 4) xanthan and glycerol, or 5) CMC50k and glycerol, or 6) xanthan, Solutol HS15 and glycerol.

In order to timely disappear, the carrier will disintegrate, and particularly dissolve - by having a suitable solubility in water at human body temperature (37°C) - or disassociate by any other biological mode. Water-free carriers of the present invention preferably dissolve at human body temperature (37°C) in a physiological buffer (e.g. phosphate buffer solution, PBS).

Preferred water-free carriers are characterized by the following properties: In a first aspect, the water-free carrier is selected so as to have a dissolution time, in vivo, of less than a week, preferably less than 3 days, and more preferably less than a day, wherein preferably 10 - 100%, 10 - 90 %, 10 - 80 %, 10 - 70 %, 10 - 60 %, 10 - 50 %, more preferably 50 %, 60 %, 70 %, 80 %, 90 % or 100 % are dissolved. More preferably, this dissolution time is fewer than twelve hours, six hours, three hours, two hours, one hour, or 30 minutes. In a preferred embodiment 10 - 100%, 10 - 90 %, 10 - 80 %, 10 - 70 %, 10 - 60 %, 10 - 50 %, more preferably 50 %, 60 %, 70 %, 80 %, 90 % or 100 % of the carrier are dissolved in the dissolution time.

In terms of dissolution rate, during a surgical operation, it is preferred if the water-free carrier has a minimum dissolution time that allows the surgeon to shape or inject the material and suture the wound without the risk of particle dispersion. Thereafter, the faster the dissolution, the more beneficial this can be to bone formation. Water-free carriers based on glycerol, poloxamer, polyethylene glycol, CMC, xanthan, and/or Solutol^{®} HS 15 are preferred in this respect. In this respect a dissolution onset, in vivo, of at least one hour is preferred. All in all, it is preferred if the onset of dissolution occurs within 1-3 hours, and the completion of dissolution occurs within 3 hours to a day (24 hours), and preferably within 6-12 hours, when 10 - 100%, 10 - 90 %, 10 - 80 %, 10 - 70 %, 10 - 60 %, 10 - 50 %, more preferably 50 %, 60 %, 70 %, 80 %, 90 % or 100 % of the carrier are dissolved.

The carrier is selected so as to be water-free so that the microstructure surface of the osteoinductive particles does not bear potential dissolution/precipitation phenomena that can lead to surface property changes, and thus, changes of characteristic properties such as osteoinductivity. All in all, the carrier is water-free so that the osteoinductivity is retained. Thus, the use of a water-free carrier allows longer shelf-life of premixed putties, which are osteoinductive, than any other type of carrier.

In another aspect, the water-free carrier is selected so as, when combined with calcium phosphate particles, which are preferably osteoinductive, to have a complete dissolution time upon standardized measurement in a phosphate buffer solution (PBS)1 or in a physiological saline solution, at 37°C within a week, preferably within 3 days, and more preferably within a day, most preferably within 1 to 12 hours. The standardized measurement in accordance with the invention is done on a cylinder of given volume (1.0 cc) in which particles, which are preferably osteoinductive, are dispersed in a given ratio of calcium phosphate particles to water-free carrier. In this test, the onset of dissolution is observed by visual inspection of the cylinder, as the onset of dissolution is evidenced by a change in the bulk shape. Complete dissolution, as defined in this test, occurs when the entire volume of calcium phosphate particles are visibly observed to be freely dispersed into solution, with no discernible bulk shape or organization whatsoever, i.e., a flat layer of loose calcium phosphate particles. According to the invention, it is preferred if the dissolution starts within 0.5-6 hours, preferably 1-6 hours, most preferably 1-3 hours, and is complete within 1-24 hours, 3-24 hours, or 6-24 hours, preferably 1-12, 3-12, 6-12 hours, more preferably within 1-8, 2-8, 3- 8 or 6-8 hours after surgical administration.

The dissolution time of the putty, which is preferably osteoinductive, in a buffer, e.g., physiologically acceptable fluids, particularly water, saline or PBS is preferably 1 to 720 min, 1 to 180 min., 1 to 120 min., 1 to 100 min., 1 to 60 min., 5 to 60 min., 10 to 60 min., 20 to 60 min., or 30 to 60 min, preferably the dissolution time is <7200 min, < 180 min., < 120 min., < 100 min., <60 min., < 30 min., or < 5 min after submersion in a buffer.

In yet another aspect, the carrier is selected so as to have a Dimensional Stability Life (DSL) of at most a week, preferably at most 3 days, and more preferably at most a day. DSL refers to the time period during which the polymer, in a human body temperature environment, is not substantially dissolved or disassociate, and is determined by the same visual inspection of the same standard cylinders mentioned above. The assessment is different in that the inspection is not of the extent of particles disassociated from the cylinder, but of the dimensions of the cylinder. The DSL is preferably similar to the aforementioned onset of dissolution, and thus, a DSL having a dissolution time of at most 1-24, 1-12, 1-6 hours, preferably at most 1-3 hours after placement in a human body temperature environment is preferred.

Water-free carriers, by which the foregoing values can well be obtained, are selected from the group comprising: 1) xanthan, dextran, starch and glycerol, or 2) CMC7H, PEG4k, PEG400 and glycerol, or 3) xanthan, dextran and Solutol HS15, or 4) xanthan and glycerol, or 5) CMC50k and glycerol, or 6) xanthan, Solutol HS15 and glycerol.

In a preferred embodiment, a water-free carrier of the present invention, when combined with calcium phosphate particles has handling characteristics that are described as cohesive, moderately adhesive, and reasonably stiff similar to that of chewing gum. In a preferred embodiment, the water-free carrier containing calcium phosphate particles can be handled, shaped, and manipulated without excessive residue or stickiness imparted to surgical gloves. In another preferred embodiment, a water-free carrier combined with calcium phosphate particles is injectable through a standard syringe. In a preferred embodiment, the water-free carrier combined with calcium phosphate particles can withstand surgical irrigation with saline or water, typical of an applicable surgical procedure. Functionally, these water-free carriers offer the advantage to be (i) formable, malleable, kneadable, moldable, and/or injectable, i.e., able to deform or flow under compressive stress, (ii) sufficiently cohesive and sticky allowing to physically bind particles and to adhere to the surrounding bone, and (iii) that they are water-soluble and fast-degrading at 37°C but temporarily resistant to typical surgical irrigation. A water-free carrier or water-free carrier combination of the invention, alone or in combination with porous calcium phosphate, which is preferably osteoinductive, shows these advantageous handling characteristics based on their "tunable" chemical composition. Specifically, a water-free carrier of the present invention can be made to be ideally moldable or ideally injectable by varying the ratios of the comprised chemical constituents, curing time, and/or curing temperature - for example, 1% (w/v) CMC suspended in glycerol is used as an injectable whereas 15% (w/v) CMC suspended in glycerol is used as a putty; moreover, 2% (w/v) xanthan suspended in glycerol and cured for 45 min at 80°C is used as an injectable whereas the same formulation cured for 1.5 hr at 98.5°C is used as a putty.

### Manufacture

The surface-microporous calcium phosphate materials, which are preferably osteoinductive, can be prepared in accordance with, e.g., the aforementioned references US 6,511,510 and WO 2007/94672. Thus, one method involves sintering a ceramic material under such conditions that a biomaterial, which is preferably osteoinductive, as described above is obtained. The ceramic material is, before the sintering, in a calcined state. The sintering is preferably performed at a temperature between 1000 and 1275°C, treated with an aqueous solution of an organic acid and washed to remove the acid. More preferably, the sintering is carried out at a temperature between 1150 and 1250°C. The duration of the sintering step may suitably be chosen between 6 and 10 hours, preferably between 7 and 9 hours. It has further been found advantageous to perform the sintering while the ceramic material is submersed in a powder of the ceramic material. This beneficially affects the reactivity of the surface of the material, and consequently also the bioactivity for example based on dissolution, re-precipitation etc. After the sintering, the material is preferably ground with sandpaper, such as Si-C sandpaper, to remove chemical surface impurities. Subsequently, the material is treated with an aqueous solution of an acid. Suitable acids in this regard are any etching acids, i.e., any acid which leads to a slight dissolution of the calcium phosphate based material. The use of the following acids, for example, has been found to lead to extremely favorable results: maleic acid, hydrochloric acid, phosphoric acid, and combinations thereof. The concentration of the acid in the solution is preferably chosen such that the pH of the solution lies between 0 and 4, more preferably between 1 and 3. After the acid treatment, which preferably lasts between 3 and 15 minutes, the ceramic material is washed to remove the acid. The washing may suitably be performed using ethanol, water or a combination thereof.

The ceramic material may be prepared by a method for producing a porous calcium phosphate ceramic, which is osteoinductive, comprising an aqueous slurry of a calcium phosphate powder having a particle size of 1.0-8.0 µm, preferably of 2.0-4.0 µm, a foaming agent and optionally a porogenic agent in water; subjecting the slurry to conditions which cause foaming of said slurry; drying the resultant foamed slurry, optionally removing the porogenic agent, to provide a porous green body and sintering the porous green body at a temperature between 1050°C and 1150°C to provide the porous sintered calcium phosphate; and optionally milling the sintered calcium phosphate to particles and collecting the particles having a particle size ranging from about 40 to about 1500 µm. The method further includes the step of milling the sintered calcium phosphate to particles, wherein the particles are collected by using sieves, preferably 45 and 500 µm sieves to provide a microparticle fraction of 45-500 µm, and more preferably 45 and 300 µm sieves to provide a microparticle fraction of 45-300 µm, and most preferably 45 and 150 µm sieves to provide a microparticle fraction of 45-150 µm to prepare injectable formulations. Most preferably, the microparticles have a size of 45-106 µm, 106-212 µm, or 212-300 µm. In a preferred embodiment, the method further includes the step of milling the sintered calcium phosphate to particles, wherein the particles are collected by using sieves, preferably 150- 500 µm, 500-1000 µm and 1000- 2000 µm sieves to provide a microparticle fraction of respectively 150 -500 µm, 500-1000 µm and 1000- 2000 µm to prepare moldable putty formulations or injectables.

The calcium phosphate powder may be a powder that is composed of crystals having a crystal size between 0.01 and 1 µm, preferably between 0.05 and 0.5 µm. The foaming agent may be hydrogen peroxide. The porogenic agent may comprise naphthalene particles, wherein the porogenic agent is removed by evaporation at 80-110°C.

The porogenic agent may comprise wax particles, wherein the porogenic agent is removed by foaming at 50-70°C followed by pre-sintering at 980-1020°C. Said conditions which cause foaming of said slurry comprise heating of the slurry to about 50-70°C.

The dried and foamed slurry is sintered at a temperature of 1050-1100°C in the case of TCP, more preferably 1050-1075°C, or at a temperature of 1100-1150°C in the case of HA and/or BCP. The calcium phosphate particles are mixed with the water-free carrier in a volumetric ratio of 1:10 to 10:1 , preferably in a ratio 2:5 to 5:2, more preferably in a ratio comprised between 1:2 to 2:1 , and most preferably 3:2 for the manufacturing of material, which is osteoinductive. The water-free carrier comprises or consists of a blend of water-free carriers as defined in claim 1.

In a further preferred embodiment (see Examples 4 and 5), the water-free carriers are made according to the same general procedure:
The masses of dry powder components are measured by mass or volume and thoroughly mixed into a non-aqueous solvent at room temperature or warmer for example 22°C to 98.5°C.

Synthetic polymers such as PEG, poloxamers, or surfactants are melted and then combined into this mixture resulting in a complete carrier solution.

The carrier solution is heated at a specific temperature, for example XDS which is heated at 98.5°C, for a specific time, for example 45 min, with or without stirring.

The carrier solution is removed from heat and cooled naturally to room temperature or rapidly with refrigeration, with or without stirring. They are then stored for combination at room temperature.

Calcium phosphate particles, e.g., TCP or BCP, should not be stored with water- containing carriers. Preferably, material of the present invention, which is osteoinductive e.g., putty presentations or injectable material are prepared as - premixed putty or injectable material, which is osteoinductive: a blend of particles, which is osteoinductive, and a water-free water-soluble carrier in an appropriate container (syringe or a vial),
- dry putty blend or injectable material, which is preferably osteoinductive, i.e., one container combining particles, which is preferably osteoinductive, with the water-free carrier under water-free powder form (i.e., lyophilized). The putty or injectable material is rehydrated in the operating suite using sterile saline or patient's blood, bone marrow, or any other body fluid.

### Uses

The materials of the invention can be used in any application where, in an animal and preferably a human, bone growth is desired. Particularly, the invention finds usage as synthetic bone void filler. The preferred target population is individuals with bony defects resulting from trauma or surgery. The preferred anatomical sites are: bony voids or gaps of the skeletal system, i.e., the extremities, spine, and pelvis, cranio-maxillofacial areas. Accordingly, the injectable material or putty is intended for use as bone void filler for voids and gaps in nonbearing bone structures. It is indicated for use in the treatment of surgically created osseous defects or osseous defects resulting from traumatic injury to the bone. The injectable material or putty is intended to be packed into bony voids or gaps of the skeletal system (i.e., extremities, spine, and pelvis).

With reference to the properties of the present surface-microporous calcium phosphates, such as osteoinductivity, adding active materials to promote bone growth are not necessary. Any such active materials can be used, examples of which include biologically active agents, growth factors and hormones, cells such as stem cells, osteogenic cells, and osteoprogenitor cells.

The invention will be further explained hereinafter with reference to the following Examples and Figures. The Examples and Figures are illustrative only and allow extrapolation to the results of in vivo, ex vivo, and in vitro use, and do not limit the invention. Those examples which do not comprise the water-free carriers as defined in claim 1 are not covered by the claims and are for reference only. Examples 1 to 3 are not part of the present invention.
Figure 1. Representative histological sections show abundant bone formation in the BCP samples of 212-300 µm (A), 106-212 µm (B) and 45-106 µm (C) while no bone in samples of smaller than 45 µm (D) (un-decalcified sections stained with methylene blue and basic fuchsin; red: bone and dark: implanted materials).
Figure 2. Histomorphometrical data showing the area percentage of bone in the whole sample, the area percentage of bone in the available space, and the volume of bone in samples of BCP particles with different particle size.
Figure 3: Representative histological cross-sections of critical-size iliac wing defects implanted with P84/F87 ceramic putty, C: P85/PEG4000 ceramic putty, and TCP particles alone (control).

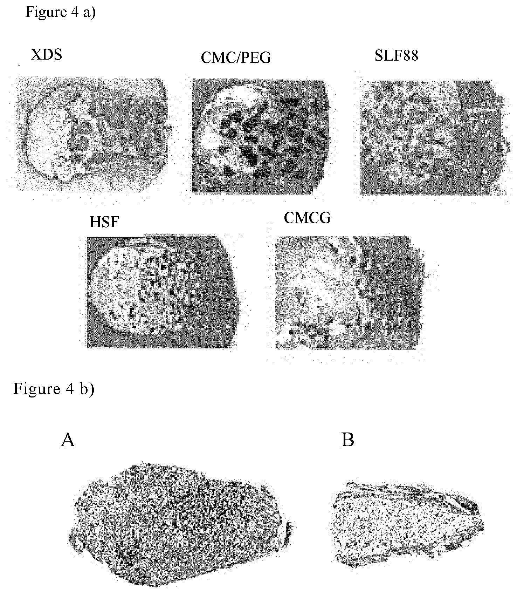

Figure 4: Representative histological cross-section of canine femoral defects (Fig. 4a) and intramuscular implants (Fig. 4b) containing osteoinductive water-free putties and injectables at 12 weeks, stained with basic fuchsin and methylene blue to visualize bone formation. Fig. 4a) Femoral defects show TCP granules (tan, black) were retained in the defects surrounded by extensive bone (pink) regeneration. Fig. 4b) Intramuscular implant sections of CMCG carrier containing osteoinductive TCP granules (150-500 µm) (A) or TCP granules (150-500 µm) alone (B). Extensive bone formation (pink) throughout the CMCG implant (A) demonstrates the osteoinductivity of TCP granules comprised in such water-free carrier.

### Examples

Reference **Example 1:** Osteoinduction of injectable ceramic particles in vivo Ceramic particles (biphasic calcium phosphate ceramic, BCP) smaller than 45 µm, 45-106 µm, 106-212 µm and 212-300 µm were prepared with sieves, cleaned ultrasonically, dried and then sterilized.

Ceramic particles (1000±10mg per sample) were implanted in the paraspinal muscle of 8 mongrel dogs for 12 weeks to evaluate inductive bone formation. With the permission of the local animal care committee, surgical operation was performed on 8 skeletally mature mongrel dogs (male, 10-15 kg) obtained from a local stock breeder. After anaesthetizing the dogs by an intra-abdominal injection of sodium pentobarbital (30 mg/kg body weight), the back was shaved and the skin was cleaned with iodine. Then a longitudinal incision was made and the paraspinal muscle exposed by blunt separation. Longitudinal muscle incisions were subsequently made by scalpel and muscle pouches were created by blunt separation. Ceramic particles were then pushed into the muscle pouches with the assistance of 2 ml syringe, and the wound was closed in layers using silk sutures. Four separate muscle pockets at least 2 cm apart were created in each side of the paraspinal muscle, and in each pocket one sample was implanted. Following surgery, each dog received penicillin intramuscularly for 3 consecutive days to prevent infection.

Twelve weeks after implantation, the animals were sacrificed and implants were harvested with surrounding tissues and immediately fixed in 4% buffered formaldehyde solution (pH=7.4). After washing with phosphate buffer solution (PBS), soft tissues surrounding the explants were carefully trimmed and the volume of the explants (Ve) was measured by displacement of water. Then the explants were dehydrated in a series of alcohol solutions (70%, 85%, 90%, 95% and 100%x2) and embedded in methyl methacrylate (MMA). Thin sections (10-20 micrometer) were made across the middle of the samples with a diamond saw (SP1600, Leica, Germany). Sections were stained with methylene blue and basic fuchsin.

Histomorphometry was performed using Adobe Photoshop software. First, the entire sample was selected as region of interest (ROI) and the pixels of the region were read (ROI), then both BCP and mineralized bone were pseudo-coloured and the pixels of BCP and bone were read as M and B respectively. The area percentage of material in the samples (Mi% for implants and Me% for explants) was calculated as M*100/ROI, percentage of available space (porosity, Pi% for implants and Pe% for explants) was calculated as (ROI-M)* 100/ROI, percentage of bone in the available space of the explants (BP%) was calculated as B* 100/(ROI-M) and the area percentage of bone in the whole sample (BROI%) was calculated as B*100/ROI.

The following data were available for analysis and comparison: Vi (Volume of the implants), Ve (volume of the explants), Mi% (area percentage of BCP in the implants), Me% (area percentage of BCP in the explants), Pi% (area percentage of available space in the implants), Pe% (area percentage of available space in the explants), BP% (area percentage of bone in the available space of the explants) and BROI% (area percentage of bone in the explants). The volume of BCP materials in the implants can be roughly determined as Vi*Mi, the volume of BCP in the explants can be determined as Ve*Me and the volume of bone in the explants can be roughly determined as Ve*BROI%.

### Results

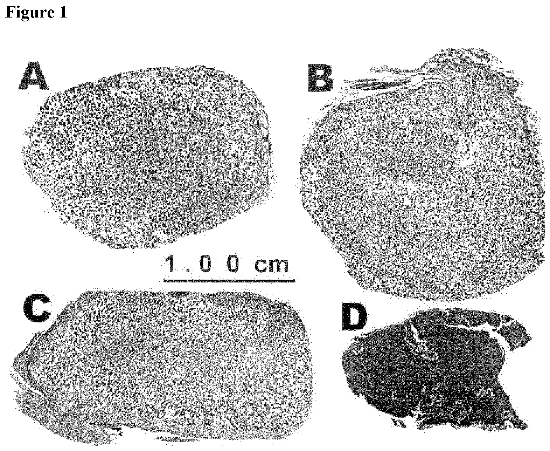

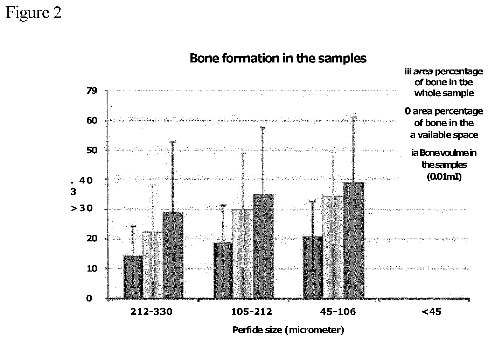

Bone was formed in all implants containing BCP particles larger than 45 µm, while no bone was formed in those containing BCP particles smaller than 45 µm (Figure 1 and 2). No significant difference was found in bone formation between 212-300 µm particles, 106- 212 µm particles and 45-106 µm particles.

### Discussion

It is shown in this study that particle size has influence of osteoinduction of CaP ceramics.

The particles larger than 45 µm demonstrated osteoinductive properties and thus can be used in developing osteoinductive ceramic materials with suitable polymeric carriers.

### Reference

### Example 2: Formulation of water-free carriers with tailored in vitro degradation characteristics

In this study, various water-free carrier formulations were developed with the aim of obtaining tailored moldability, injectability, and dissolution kinetics. These formulations were made using raw materials with proven biocompatibility, medical use history, rapid dissolvability, and lubricating effect, as summarized below:
- Polyethylene glycol (PEG): PEG400, PEG1000, PEG4000, PEG20000 (e.g., Merck Chemical Industry),
- Pluronic^{®} : P65, P84, P85, F87, F88, F98 (e.g., BASF Benelux) and F127 (e.g., Sigma),
- Polyol: Glycerol (e.g., Merck Chemical Industry),
- Emulsifier: Soya Lecithin (e.g., AMD Special Ingredients),
- Carbohydrate: Sucrose (e.g., Sigma Aldrich).

Formulations were made by mixing two of the above components by volume. Preliminary formulations were screened by their suitability as a particle binder, based on a qualitative handling assessment. Only the positively evaluated carriers were thereafter blended with TCP particles (500-1000 µm), in a volumetric ratio of 2:3, carrier to TCP. These resulting ceramic putty materials were then scored on the basis of handling, cohesiveness, and stickiness. Finally, the dissolution kinetics of each formulation was evaluated follows: a 1 cc cylinder of the ceramic material was immersed in 8 cc phosphate buffered saline (PBS) at 37°C to mimic physiological body fluid. The samples were then visually monitored for dissolution, defined here as when calcium phosphate particles freely disassociate from the bulk material, forming an amorphous layer of particles at the bottom of the vessel. The time required for -75% dissolution was recorded.

### Results

Blending a single component, i.e. Pluronic^{®}, PEG, glycerol, soya lecithin or sucrose polymer with TCP particles did not lead to cohesive and moldable putty formulations, while blending two of these components potentially led to cohesive and moldable formulations. The best handling, cohesiveness and stickiness scores were observed in the five ceramic materials summarized in the table below:

**Table 1: Selected water -free ceramic materials**

| **Compound 1 (% vol)** | **Compound 2 (% vol)** | **Carrier/TCP** | **Remarks** | **Time for >75% Dissolution** |
|---|---|---|---|---|
| P84 (75%) | F87 (25%) | 2:3 | Moldable, very cohesive | <120 min |
| P85 (60%) | PEG 1000 (40%) | 2:3 | Moldable, uniform, oily, very cohesive | <60 min |
| P85 (90%) | PEG 4000 (10%) | 2:3 | Moldable, uniform, very cohesive | <60 min |
| PEG 4000 (56%) | PEG400 (44%) | 2:3 | Moldable | < 30 min |
| F88 (50%) | Soya Lecithin (50%) | 2:3 | Moldable | <12 hr |

### Discussion

The combination of two water-free components has a substantial effect on the carrier characteristics, although not all combinations make for suitable carriers. The materials summarized in Table 1 comprise the best performing carriers developed in this research. These water-free carriers are preferred for combination with microstructured CaP particles because they substantially prolong the functional shelf-life as compared to water- containing carriers.

### Reference Example 3: In vivo efficacy of three different water-free carriers

Various water-free carriers were prepared and combined with TCP granules to form putties as described in Example 2. These formulations were blindly evaluated on the basis of handling characteristics and two out of five were selected for in vivo implantation. These putties were implanted in critical sized defects (19 mm) in the iliac wings of goats for a period of 16 weeks. After explanation, the samples were fixed in formalin, dehydrated using ethanol, and embedded in methyl methacrylate. Calcified sections were made and stained with methylene blue and basic fuchsin solutions to visualize bone formation. Bone formation in the putty samples was compared to that of TCP granules with no carrier - also implanted in the same goats as a control. The experimental design is summarized in Table 2, below.

**Table 2: Study design summary**

| **Sample formulation** | **# of test subjects implanted** | **Location of implant** |
|---|---|---|
| **75% P84, 25% F87 putty** | **2** | **Iliac crest** |
| **90% P85, 10% PEG4000 putty** | **1** | **Iliac crest** |
| **TCP 500-1000 µm** | **2** | **Iliac crest** |

### Results

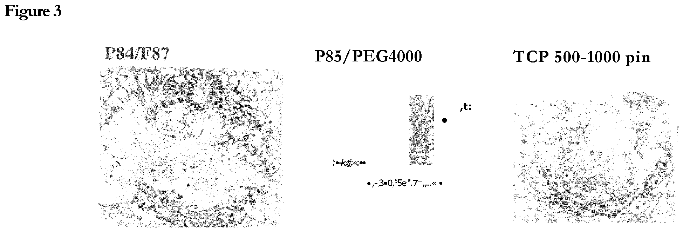

Histological sections taken from the middle of the iliac wing defect explants showed bone formation in all implants. For both water-free formulations, new bone formation was in contact with the comprised TCP particles, indicating genuine integration of the materials into the bony defect. Bone formation in P85/PEG4000 (Figure 3) and P84/F87 (Figure 4 B) samples was comparable to TCP 500-1000 µm granules alone (Figure 3).

### Discussion

The quality of bone formation was comparable for all treatment groups - i.e., new bone formation was in direct contact with the CaP particles. This indicates that the tested water-free carriers do not interfere with new bone formation. Moreover, no residual carrier remnants were observed, indicating that these water-free formulations are readily disintegrated in vivo, as observed in vitro previously. In summary, these results support the conclusion that these water-free carriers are useful in delivering osteoinductive CaP materials for bone defect repair.

### Example 4: Formulation of water-free osteoinductive putties

A variety of both naturally occurring and synthetic material components were selected to construct biocompatible water-free, moldable carriers with tuneable handling characteristics and specific degradation kinetics. They are generally categorized as either solvents or thickeners:

### Solvents:

- Polyethylene glycol, mw=400 (Merck) ("PEG400")
- Glycerol (Sigma) ("Gly")

### Thickeners:

- Polyethylene glycol, mw=4,000 (Fluka) ("PEG4k")
- Dextran, mw=40,000 (Pharmacosmos) ("Dex")
- Xanthan XGF FNHV (Jungbunzlauer) ("Xan")
- Blanose^{®} sodium carboxymethyl cellulose, 7H4XF PH (Hercules) ("CMC7H") Starch, soluble (Sigma)
- Soya lecithin (AMD)
- Pluronic^{®} F88 (BASF) ("F88")
- Solutol^{®} HS 15 (BASF) ("HS15")

Multiple formulations were prepared using different combinations of these components to develop moldable carriers with different defined stiffness, homogeneity, and viscosity. The formulations were made according to the following general procedure: Solvent(s) and thickener(s) were measured by volume and mass, respectively, and then blended until homogeneous.

The carrier formulations useful as moldable particle binders were then combined with TCP particles (500-1000 µm). Such carrier formulations are for example: xanthan, dextran, starch, and glycerol (XDS); CMC7H, PEG4k, PEG400, and glycerol (CMC/PEG); soya lecithin and F88 (SLF88); and xanthan, dextran, and Solutol^{®} HS 15 (XDHS). Carriers and TCP particles were mixed until homogeneous in volumetric ratios ranging from 1 :2 to 3:4, carrier to particles. These were then evaluated as moldable ceramic putties and scored on their handling characteristics - specifically, cohesiveness, stickiness, and ductility.

To evaluate the dissolution kinetics of these putties - for example: XDS, CMC/PEG, and SLF88 - they were submerged in PBS at 37°C (1 :10 v/v) and visually monitored for dissolution as in Example 2.

### Results

Together, the handling characteristics and dissolution data provided a basis to refine the body of water-free carriers developed to only those that met the desired requirements, which are for example moldability and water solubility within 2 days (Table 3).

**Table 3: Summary of selected moldable water-free ceramic materials**

| **Putty Formulations** | **Composition** | | **Complete dissolution** | **Characteristics** |
|---|---|---|---|---|
| | **Solvent** | **Thickener** | | |
| **XDS** | Gly | 60% Dex, 10% Starch, 2.5% Xan (w/v) | < 2 hours | Ductile, cohesive, moldable, sticky |
| **CMC/PEG** | PEG400, Gly (1:1, v/v) | 15% CMC7H, 25% PEG 4k (w/v) | < 21 hours | Cohesive, ductile, moldable, highly |
| **SLF88** | Soya Lecithin | 50% F88 (w/w) | < 21 hours | Moldable |
| **XDHS** | HS 15, Gly (1:9, v/v) | 2.5% Xan, 70% Dex (w/v) | < 18 hours | Ductile, cohesive, moldable |

### Discussion

Using a wide variety of components as listed in Table3, moldable osteoinductive putties - for example, XDS (xanthan, dextran, starch and glycerol), CMC/PEG (CMC, PEG 400, PEG 4k, and glycerol), SLF88 (soya lecithin and Plu F88), and XDHS were developed with customized handling characteristics to dissolve within two days. Being water-free, these putties provide extended shelf life without causing hydrolytic degradation of the TCP particles.

### Example 5: Formulation of water-free osteoinductive iniectables

A variety of both naturally occurring and synthetic material components were selected to construct biocompatible, water-free, injectable carriers with desired handling characteristics and specific degradation kinetics: - CeKol 50000 carboxymethyl cellulose (CP Kelco) ("CMC50k")
- Glycerol (Sigma) ("Gly")
- Lutrol^{®} F 127 (BASF) ("F127")
- Solutol^{®} HS 15 (BASF) ("HS15")
- Xanthan XGF FNHV (Jungbunzlauer) ("Xan")

Multiple formulations were prepared using a combination of the different components to develop injectable carriers with optimal stiffness, homogeneity, and viscosity for extrusion through a luer tip syringe. The formulations were made following the same general procedure: Component quantities were measured by volume or mass and then blended until homogeneous.

These carrier formulations useful as injectable particle binders were then combined with TCP particles (150-500 µm). For example, such carrier formulations are comprised of xanthan and glycerol (XG); F127 and HS15 (HSF); CMC50k and glycerol (CMCG); and xanthan, Solutol^{®} HS 15, and glycerol (XHS). Carriers and TCP particles were mixed until homogeneous in the volumetric ratios ranging from 0.6:1 to 1:1 , carrier to particles, and then transferred to a variety of luer tip syringes. The injectable particle binders were then evaluated on the basis of their handling characteristics - for example, cohesiveness, stickiness, and ease of extrusion.

To evaluate their dissolution characteristics, these putties - for example: XG, HSF, and CMCG - were submerged in PBS at 37°C (1:10 v/v) and visually monitored for dissolution as in Example 2.

### Results

Together, the handling characteristics and dissolution data provided a basis to refine the body of water-free carriers developed to only those that met the desired requirements, for example: injectability and water solubility within two days. These formulations are summarized in Table 4.

**Table 4: Summary of injectable water-free ceramic materials**

| **Injectable Formulations** | **Composition** | | **Complete dissolution** | **Characteristics** |
|---|---|---|---|---|
| **XG** | 1% Xan, Gly (w/v) | | < 1 hr | Flowable, sticky |
| **HSF** | 42% F127, 58% HS15 (w/w) | | < 5 hr | Flowable, sticky |
| **CMCG** | 5% CMC, Gly (w/w) | | < 1 hr | Flowable |
| **XHS** | HS 15, Gly (2:3, v/v) | 1% Xan (w/v) | < 12 hr | Flowable, moldable, sticky |

### Discussion

Using a variety of components listed in Table 4, osteoinductive injectables - for example, XG (xanthan and glycerol), HSF (Solutol^{®} HS 15 and Plu F127), CMCG (CMC and glycerol), and XHS (xanthan, Solutol^{®} HS 15, and glycerol) - were developed with customized handling characteristics to dissolve within two days. Being water-free, these injectables provide extended shelf life without hydrolytic degradation of the TCP particles.

### Example 6: In vivo bone formation of water-free osteoinductive putties and injectables

Osteoinductive water-free materials in the form of putty or injectable containing TCP particles (previously described in Examples 4 and 5) were implanted in 8 male dogs to evaluate their bone forming potential *in vivo.* The materials were implanted in both osseous and non-osseous sites - the femur and paraspinal muscles, respectively. Femoral defects 5 mm in diameter were filled with less than 0.5 cc of material test sample and 1 cc samples were implanted in the muscle. Tricalcium phosphate (TCP) particles (500-1000 and 150-500 µm) alone were implanted as control. The implants were harvested after 12 weeks and prepared for histological evaluation. Test samples were stained with methylene blue and fuchsin to visualize bone formation.

Additionally, carrier formulations were evaluated on the time required for complete dissolution in phosphate buffered saline (PBS), analogous to physiological body fluid. One cc samples of each carrier formulation, without calcium phosphate granules, were submersed in 8 cc PBS and stored at 37°C. The samples were visually monitored for complete dissolution - here defined as when no discernible bulk shape, form, or fragment of the carrier is visible in PBS and the carrier-PBS mixture is visibly homogenous. The time for complete dissolution was recorded.

**Table 5: Summary of femoral defect implants**

| **Formulation** | **Carrier:TCP (v/v)** | **TCP particle size (µm)** | **Form** |
|---|---|---|---|
| **XDS (Example 4)** | 2:3 | 500-1000 | Putty |
| **CMC/PEG (Example 4)** | 2:3 | 500-1000 | Putty |
| **SLF88 (Example 4)** | 2:3 | 500-1000 | Putty |
| **HSF (Example 50)** | 1:1 | 150-500 | Injectable |
| **CMCG (Example 5)** | 1:1 | 150-500 | Injectable |

**Table 6: Summary of intramuscular implants**

| **Formulation** | **Carrier:TCP (v/v)** | **TCP particle size (µm)** | **Form** |
|---|---|---|---|
| **XDS (Example 4)** | 2:3 | 500-1000 | Putty |
| **CMC/PEG (Example 4)** | 2:3 | 500-1000 | Putty |
| **XG (Example 5)** | 1:1 | 150-500 | Injectable |
| **HSF (Example 5)** | 1:1 | 150-500 | Injectable |
| **CMCG (Example 5)** | 1:1 | 150-500 | Injectable |

Histological staining of the femoral implants (Figure 4 a)) show that TCP particles were retained by the water-free carriers in the defects, as intended. Moreover, extensive bone regeneration was apparent throughout the defects. Notably, no residual carrier material was observed in any of the implant sections examined.

Staining of the intramuscular implants (Figure 4b)) demonstrate the retention of osteoinductivity by TCP granules comprised in water-free carriers, as evidenced by ectopic bone formation in the muscle (CMCG depicted as an example) in all formulations except for SLF88.

*In vitro* dissolution tests of the carriers alone showed that SLF88 was the only carrier to not fully dissolve within two days *in vitro.*

### Discussion

The results of this study demonstrate the utility and efficacy of applying water-free carriers, such as the ones developed herein, to the delivery of osteoinductive ceramics. These carriers can handily deliver ceramic materials to the defect site and retain them in situ for optimal bone repair. Notably, the in vitro dissolution data illustrated an important relationship between bone forming potential and in vitro dissolution time of carriers used in osteoinductive putties. Specifically, fast-dissolving water-free carriers are preferred to slow-dissolving carriers, as slow dissolution may inhibit osteoinduction of the comprised microstructured calcium phosphate particles. However, a slow-dissolving carrier (e.g., SLF88) may be 'tuned' to be fast-dissolving by modifying carrier chemical composition (e.g., concentration, molecular weight), curing temperature (e.g., cure at 60°C rather than 98.5°C), and curing time (e.g., 45 min rather than 90 min).

## Claims

1. An osteoinductive ceramic material comprising:
porous calcium phosphate having a surface microporosity and a water-free carrier, wherein the water-free carrier comprises: 1) xanthan, dextran, starch and glycerol, or 2) CMC7H, PEG4k, PEG400 and glycerol, or 3) xanthan, dextran and Solutol HS15, or 4) xanthan and glycerol, or 5) CMC50k and glycerol, or 6) xanthan, Solutol HS15 and glycerol,
wherein the porous calcium phosphate comprises macropores and micropores at least present in the surface of the macropores, and the porous calcium phosphate is in form of microparticles having a particle size of at least 45 µm.

2. The osteoinductive ceramic material of claim 1, wherein the porous calcium phosphate is in a form of microparticles having a particle size ranging from about 45 to about 1500 µm.

3. The osteoinductive ceramic material of any one of claims 1-2, wherein the porous calcium phosphate is in a form of microparticles having a particle size ranging from 45-106 µm, 106-212 µm, or 212-300 µm.

4. The osteoinductive ceramic material of any one of claims 1-3, wherein the water-free carrier is formable, moldable, malleable, and/or injectable.

5. The osteoinductive ceramic material of any one of claims 1-6, wherein the water-free carrier has a dissolution time of less than 1 day or less than 3 days.

6. The osteoinductive ceramic material of claim 5, wherein 50% of the water-free carrier is dissolved.

7. The osteoinductive ceramic material of any one of claims 1-6, wherein the water-free carrier comprises a polymer that has a Dimensional Stability Life (DSL), which is the time period during which the polymer, in a human body temperature environment is not substantially dissolved or disassociated determined by visual inspection of at most a day or at most 3 days.

8. The osteoinductive ceramic material of any one of claims 1-7, wherein the water-free carrier is water soluble.

9. The osteoinductive ceramic material of any one of claims 1-8, wherein the porous calcium phosphate has a total porosity of 20 to 90%, wherein the macropores are present having a size of from 0.1 to 1.5 mm, and wherein the micropores are present having a size of from 0.05 to 20 µm.

10. The osteoinductive ceramic material of any one of claims 1-9 having an average grain size in a range of 0.1-1.50 µm, a porosity comprising micropores in a size range of 0.1-1.50 µm, and having a surface area percentage of micropores in a range of 10-40%.

11. The osteoinductive ceramic material of any one of claims 1-10, wherein the porous calcium phosphate comprises interconnected macropores having a size of from 0.2 to 1.0 mm.

12. The osteoinductive ceramic material of any one of claims 1-11, wherein the micropores have a size of from 0.1 to 1.5 µm.

13. The osteoinductive ceramic material of any one of claims 1-12, for use in promoting bone formation in a subject in need thereof, wherein a biocompatible carrier is combined with the osteoinductive ceramic material.

14. The osteoinductive ceramic material of any one of claims 1-12, wherein the water-free carrier is substantially fully dissolved or disassociated before completion of bone formation or onset of bone formation.

## Patentansprüche

1. Osteoinduktives Keramikmaterial, umfassend:
poröses Calciumphosphat mit einer Oberflächenmikroporosität und einem wasserfreien Träger, wobei der wasserfreie Träger umfasst: 1) Xanthan, Dextran, Stärke und Glycerin, oder 2) CMC7H, PEG4k, PEG400 und Glycerin, oder 3) Xanthan, Dextran und Solutol HS15, oder 4) Xanthan und Glycerin, oder 5) CMC50k und Glycerin, oder 6) Xanthan, Solutol HS15 und Glycerin, wobei das poröse Calciumphosphat
Makroporen und Mikroporen umfasst, die mindestens in der Oberfläche der Makroporen vorhanden sind, und das poröse Calciumphosphat in Form von Mikropartikeln mit einer Partikelgröße von mindestens 45 µm vorliegt.

2. Osteoinduktives Keramikmaterial nach Anspruch 1, wobei das poröse Calciumphosphat in Form von Mikropartikeln mit einer Partikelgröße von etwa 45 bis etwa 1500 µm vorliegt.

3. Osteoinduktives Keramikmaterial nach einem der Ansprüche 1 bis 2, wobei das poröse Calciumphosphat in einer Form von Mikropartikeln mit einer Partikelgröße von 45-106 µm, 106-212 µm oder 212-300 µm vorliegt.

4. Osteoinduktives Keramikmaterial nach einem der Ansprüche 1 bis 3, wobei der wasserfreie Träger formbar, gießbar, gestaltbar und/oder injizierbar ist.

5. Osteoinduktives Keramikmaterial nach einem der Ansprüche 1 bis 4, wobei der wasserfreie Träger eine Auflösungszeit von weniger als 1 Tag oder weniger als 3 Tage hat.

6. Osteoinduktives Keramikmaterial nach Anspruch 5, wobei 50 % des wasserfreien Trägers gelöst sind.

7. Osteoinduktives Keramikmaterial nach einem der Ansprüche 1 bis 6, wobei der wasserfreie Träger ein Polymer umfasst, das eine Dimensionsstabilitätslebensdauer (DSL) hat, das der Zeitraum ist, in dem das Polymer in einer menschlichen Körpertemperaturumgebung nicht wesentlich gelöst oder disassoziiert wird, was durch Sichtprüfung von höchstens einem Tag oder höchstens 3 Tagen bestimmt wird.

8. Osteoinduktives Keramikmaterial nach einem der Ansprüche 1 bis 7, wobei der wasserfreie Träger wasserlöslich ist.

9. Osteoinduktives Keramikmaterial nach einem der Ansprüche 1 bis 8, wobei das poröse Calciumphosphat eine Gesamtporosität von 20 bis 90 % hat, wobei die Makroporen eine Größe von 0,1 bis 1,5 mm haben und wobei die Mikroporen eine Größe von 0,05 bis 20 µm haben.

10. Osteoinduktives Keramikmaterial nach einem der Ansprüche 1 bis 9 mit einer durchschnittlichen Korngröße in einem Bereich von 0,1-1,50 µm, einer Porosität umfassend Mikroporen in einem Größenbereich von 0,1-1,50 µm, und mit einem Oberflächenanteil von Mikroporen in einem Bereich von 10-40 %.

11. Osteoinduktives Keramikmaterial nach einem der Ansprüche 1 bis 10, wobei das poröse Calciumphosphat miteinander verbundene Makroporen mit einer Größe von 0,2 bis 1,0 mm umfasst.

12. Osteoinduktives Keramikmaterial nach einem der Ansprüche 1 bis 11, wobei die Mikroporen eine Größe von 0,1 bis 1,5 µm haben.

13. Osteoinduktives Keramikmaterial nach einem der Ansprüche 1 bis 12, zur Verwendung bei der Förderung der Knochenbildung bei einem Subjekt, das dies benötigt, wobei ein biokompatibler Träger mit dem osteoinduktiven Keramikmaterial kombiniert ist.

14. Osteoinduktives Keramikmaterial nach einem der Ansprüche 1 bis 12, wobei der wasserfreie Träger vor Abschluss der Knochenbildung oder Beginn der Knochenbildung im Wesentlichen vollständig gelöst oder dissoziiert ist.

## Revendications

1. Matériau céramique ostéo-inducteur comprenant :
du phosphate de calcium poreux ayant une microporosité de surface et un support anhydre, lequel support anhydre comprend : 1) du xanthane, du dextrane, de l'amidon et du glycérol, ou 2) de la CMC7H, du PEG4k, du PEG400 et du glycérol, ou 3) du xanthane, du dextrane et du Solutol HS15, ou 4) du xanthane et du glycérol, ou 5) de la CMC50k et du glycérol, ou 6) du xanthane, du Solutol HS15 et du glycérol,
dans lequel le phosphate de calcium poreux comprend des macropores et des micropores au moins présents dans la surface des macropores, et le phosphate de calcium poreux est sous la forme de microparticules ayant une granulométrie d'au moins 45 µm.

2. Matériau céramique ostéo-inducteur selon la revendication 1, dans lequel le phosphate de calcium poreux est sous la forme de microparticules ayant une granulométrie située dans la plage allant d'environ 45 à environ 1500 µm.

3. Matériau céramique ostéo-inducteur selon l'une quelconque des revendications 1 et 2, dans lequel le phosphate de calcium poreux est sous la forme de microparticules ayant une granulométrie située dans la plage allant de 45 à 106 µm, de 106 à 212 µm, ou de 212 à 300 µm.

4. Matériau céramique ostéo-inducteur selon l'une quelconque des revendications 1 à 3, dans lequel le support anhydre est façonnable, moulable, malléable, et/ou injectable.

5. Matériau céramique ostéo-inducteur selon l'une quelconque des revendications 1 à 4, dans lequel le support anhydre a un temps de dissolution inférieur à 1 jour ou inférieur à 3 jours.

6. Matériau céramique ostéo-inducteur selon la revendication 5, dans lequel 50 % du support anhydre sont dissous.

7. Matériau céramique ostéo-inducteur selon l'une quelconque des revendications 1 à 6, dans lequel le support anhydre comprend un polymère qui a une durée de vie de stabilité dimensionnelle (DSL), qui est la période de temps durant laquelle le polymère, dans l'environnement de température du corps humain, n'est pas sensiblement dissous ou dissocié, déterminée par inspection visuelle, d'au plus un jour ou d'au plus 3 jours.

8. Matériau céramique ostéo-inducteur selon l'une quelconque des revendications 1 à 7, dans lequel le support anhydre est soluble dans l'eau.

9. Matériau céramique ostéo-inducteur selon l'une quelconque des revendications 1 à 8, dans lequel le phosphate de calcium poreux a une porosité totale de 20 à 90 %, dans lequel les macropores sont présents en ayant une taille de 0,1 à 1,5 mm, et dans lequel les micropores sont présents en ayant une taille de 0,05 à 20 µm.

10. Matériau céramique ostéo-inducteur selon l'une quelconque des revendications 1 à 9, ayant une grosseur de grain moyenne située dans la plage allant de 0,1 à 1,50 µm, une porosité comprenant des micropores ayant une taille située dans la plage allant de 0,1 à 1,50 µm, et ayant un pourcentage de superficie de micropores situé dans la plage allant de 10 à 40 %.

11. Matériau céramique ostéo-inducteur selon l'une quelconque des revendications 1 à 10, dans lequel le phosphate de calcium poreux comprend des macropores interconnectés ayant une taille de 0,2 à 1,0 mm.

12. Matériau céramique ostéo-inducteur selon l'une quelconque des revendications 1 à 11, dans lequel les micropores ont une taille de 0,1 à 1,5 µm.

13. Matériau céramique ostéo-inducteur selon l'une quelconque des revendications 1 à 12, pour une utilisation dans la promotion de la formation d'os chez un sujet en ayant besoin, dans lequel un support biocompatible est combiné avec le matériau céramique ostéo-inducteur.

14. Matériau céramique ostéo-inducteur selon l'une quelconque des revendications 1 à 12, dans lequel le support anhydre est pratiquement complètement dissous ou dissocié avant l'achèvement de la formation d'os ou le commencement de la formation d'os.
